**Europäisches Patentamt**

**European Patent Office**

**Office européen des brevets**

(11) Publication number : **0 369 545 B1**

# EUROPEAN PATENT SPECIFICATION

(45) Date of publication of patent specification :
**19.08.92 Bulletin 92/34**

(51) Int. Cl.⁵ : **B01J 13/00**, G01N 33/58

(21) Application number : **89202882.0**

(22) Date of filing : **14.11.89**

(54) **Process for the preparation of elemental sols.**

The file contains technical information
submitted after the application was filed and
not included in this specification

(30) Priority : **15.11.88 AU 25180/88**

(43) Date of publication of application :
**23.05.90 Bulletin 90/21**

(45) Publication of the grant of the patent :
**19.08.92 Bulletin 92/34**

(84) Designated Contracting States :
**AT BE CH DE ES FR GB GR IT LI LU NL SE**

(56) References cited :
**GB-A- 1 530 489**
**US-A- 3 697 296**
**US-A- 3 917 885**
**US-A- 4 578 181**
**LL. Dykgraaf "Grensvlak - en Kolloidchemie"**
**page 55.**

(73) Proprietor : **H.B.T. HOLLAND
BIOTECHNOLOGY B.V.
Niels Bohrweg 13
NL-2333 CA Leiden (NL)**
Proprietor : **AGROTECHNOLOGISCH
ONDERZOEKSINSTITUUT (ATO)
Haagsteeg 6
NL-6708 PM Wageningen (NL)**

(72) Inventor : **Wichers, Jan Herman
Tarthorst 703
NL-6708 JA Wageningen (NL)**
Inventor : **Van Gelder, Wilhelmus Martinus
Jozef
M.L. Kingplantsoen 2
NL-6671 BP Zetten (NL)**
Inventor : **Van Doorn, Albert Willem Jacob
Jacob Marislaan 60
NL-6813 JX Arnhem (NL)**

(74) Representative : **Smulders, Theodorus A.H.J.,
Ir. et al
Vereenigde Octrooibureaux Nieuwe Parklaan
97
NL-2587 BN 's-Gravenhage (NL)**

## Description

The invention relates to a process for the preparation of a sol of a non-metallic element selected from the group consisting of selenium and tellurium, comprising the step of treating a solution of a chemical compound, which contains said element in a reducible state, with a reducing agent.

Such a process is known both from very old and from very recent publications. More than hundred years ago, Schulze, J. Pract. Chem. (2), 32, 390, 1885, described a preparation of selenium sols (i.e. dispersions of selenium particles in an aqueous liquid; the sols may also be referred to as hydrosols) by adding $S_2$ to an aqueous solution of selenious acid ($H_2Se_3$). The sols thereby obtained are quite turbid, however, and can only be stored for a very short time. In 1902, Gutbier, Z. Anorg. Chemie 32, 52, 1902, described a preparation of tellurium sols by treating an aqueous solution of telluric acid ($H_6TeO_6$) with hydrazine hydrate. The black-colored sol thereby obtained is turbid and unstable, however. Gutbier, Z. Anorg. Chemie 32, 106, 1902, also described a preparation of selenium sols by using hydrazine hydrate as reducing agent. Brintzinger, Koll. Zeitschr. 78, 22, 1937, disclosed a process for the preparation of selenium, tellurium, gold, palladium, platinum, silver, molybdenum and tungsten sols by reduction (for example of $SeO_2$) with ascorbic acid or isoascorbic acid as a reducing agent. He obtained a slightly turbid red-colored selenium sol, a turbid yellowish-white silver sol, etc. A number of different reducing agents, such as formaldehyde, etheric solution of phosphorus, and hydroxylamine have also been proposed. Although some sols were more stable than others, most of the sols obtained were turbid and had a limited stability.

More recent publications on the preparation of sols are, for example, EP-A-0 007 654 (Akzo N.V., published Feb. 6, 1980) which describes the preparation of gold and silver sols from aqueous chloroauric acid ($HAuCl_4$) and silver nitrate ($AgNO_3$) solutions, respectively, by treatment with tri-sodium citrate as reducing agent. In general, the old methods for preparing elemental sols are still used, as appears from EP-A-0 298 368 (Abbott Laboratories, published Jan. 11, 1989) which uses the old process of treating an aqueous $SeO_2$ solution with ascorbic acid for the preparation of a selenium sol. The same applies to Dutch patent application NL-A-87.02769 and the corresponding part of EP-A-0 321 008 (H.B.T. Holland Biotechnology B.V., published June 21, 1989) which uses hydrazine hydrate as reducing agent for $SeO_2$ and ascorbic acid for the reduction of telluric acid.

As has been indicated hereabove, the known methods for preparing sols of a non-metallic element are not completely satisfactory in that they generally result in quite turbid and/or unstable sols. Moreover, most of the known preparation methods require operation at elevated temperatures and/or involve a substantial reaction time. For example, both the gold sol preparation according to EP-A-0 007 654 and the selenium sol preparation according to EP-A-0 298 368 require at least 10 to 15 minutes at the boiling point.

The invention now overcomes said disadvantages by a process for the preparation of a sol of a non-metallic element selected from the group consisting of selenium and tellurium, comprising the step of treating a solution of a chemical compound, which contains said element in a reducible state, with a reducing agent, the process according to the invention being characterized in that a borane compound is used as the reducing agent.

It should be understood that the words "a sol of a non-metallic element" refer to a dispersion (preferably an aqueous dispersion, but alcoholic dispersions and dispersions in other organic liquids are not excluded) of particles consisting of a non-metal substantially in its elemental form. Said words do not intend to exclude that the sol particles also contain a minor amount of the non-metal in the form of a chemical compound, i.e. the particles in a selenium sol may contain some selenium oxide, but most of the selenium will be present in the elemental form. Preferably at least 60%, more preferably at least 70% and most preferably substantially all of the selenium will be present in the elemental form. The same applies to tellurium sols.

As far as the borane compound is concerned, it is strongly preferred that a borane compound, selected from the group consisting of alkali metal borohydrides, alkali metal cyanoborohydrides, quaternary ammonium borohydrides and amine boranes, is used as the reducing agent. In a particularly preferred embodiment of the invention, a borane compound, selected from the group consisting of lithium borohydride ($LiBH_4$), sodium borohydride ($NaBH_4$), potassium borohydride ($KBH_4$), rubidium borohydride ($RbBH_4$), cesium borohydride ($CsBH_4$), lithium cyano borohydride ($LiBH_3CN$), sodium cyano borohydride ($NaBH_3CN$), potassium cyano borohydride ($KBH_3CN$), rubidium cyano borohydride ($RbBH_3CN$), cesium cyano borohydride ($CsBH_3CN$), ammonium borohydride ($NH_4BH_4$), tetramethylammonium borohydride $[(CH_3)_4NBH_4]$, dimethylamino borane $[(CH_3)_2NHBH_3]$, N,N-diethylaniline borane $[C_6H_5N(C_2H_5)2.BH_3]$ and pyridine borane ($C_5H_5N.BH_3$), is used as the reducing agent. It will be understood, however, that equivalent borane compounds may be used instead and that the invention is not limited to the above examples of suitable borane compounds. For example, other dialkylamino boranes than dimethylamino borane, such as diethylamino borane for example, and other amine borane complexes than N,N-diethylaniline borane and pyridine borane may be used as well.

Most preferably, however, sodium borohydride

$(NaBH_4)$, potassium borohydride $(KBH_4)$ or pyridine borane $(C_5H_5N.BH_3)$ is used as the reducing agent, as these borane compounds are easily available and have proven to give very clear and stable sols.

The present invention brings unexpected advantages over the sol preparation methods of the state of the art. A practical advantage is that the reaction takes only a few minutes at room temperature, whereas many of the prior art methods require that the reaction is carried out for at least 10 or 15 minutes at the boiling point. More significantly, the process according to the invention results in sols of much higher quality in that they are clear instead of opalescent or even turbid as most of the prior art sols, and that they have increased stability compared to sols obtained by a prior art method. For different reasons, clear sols are strongly preferred above turbid sols. The sol particles in a turbid sol usually have a more or less pronounced spongy nature, whereas the sol particles in a clear sol are more compact and more spherical in shape. As a result, the former particles contain less of the non-metallic element concerned which detracts from there visibility/discernibility (due to lower color intensity). More perfectly spherical and compact particles provide for a higher color intensity and a higher electrical charge which improves the stability of the sol. Also, a clear sol may be concentrated more easily without loss of its stability, whereas concentration of a turbid sol usually results in a decrease of the sol stability due to the occurrence of agglutination. Finally, clear sols are better suited for use in nephelometrical determinations as they cover broader ranges to distinguish clear sols and agglutinated sols.

In view of these unexpected and highly advantageous properties of sols prepared by the process of the invention, the invention also extends to sols of a non-metallic element selected from the group consisting of selenium and tellurium, which are obtained or obtainable by the process of the invention.

As is evident from the cited publications EP-A-0 007 654, EP-A-0 298 368 and NL-A-87.02769/EP-A-0 321 008, sols of metallic or non-metallic elements may be used for the labelling of a component of the reaction between a specifically-binding substance and a corresponding bindable substance. The invention extends to such a use, in particular to the use of a sol of a non-metallic element selected from the group consisting of selenium and tellurium, which is obtained or obtainable by the process according to the invention, as a label for a component of the reaction between a specifically-binding substance (such as protein, DNA, RNA, polysaccharide) and a corresponding bindable substance in a method for assaying, detecting or measuring the presence and/or quantity of a component of said reaction in a test sample.

The invention will be explained in greater detail by means of the following examples of preferred embodiments thereof. It should be noted, however, that the examples are not intended to limit the scope of the invention but merely serve to illustrate the invention.

## Example 1

0.20 g selenium oxide $(SeO_2$, Baker Chemical Company) was dissolved at room temperature in 995 ml demineralized water. The solution was constantly stirred with a magnetic bar. After stirring for 3 min, a freshly prepared solution of 0.25 g $NaBH_4$ (Chemetal) in 5 ml demi water was quickly added under vigorous stirring. While releasing $H_2$, the original clear and colorless reaction mixture obtained a clear deep orange/red color after 3 min. After that time, no further increase of color was observed.

The selenium sol was purified and eventually concentrated by dialysis. The particles had a spherical shape and an average diameter of $35 \pm 4$ nm. The sol was stable and remained clear for several days.

## Example 2

Except for using $KBH_4$ instead of $NaBH_4$, the procedure of example 1 was repeated. As a result, an identical sol was obtained in a somewhat less violent reaction.

## Example 3

0.1 g $SeO_2$ (ICN Chemicals) was dissolved in 500 ml of demineralized water. 0.125 ml borane pyridine complex (Janssen Chimica) was added under vigorous stirring at room temperature Within 5 min, a clear sol having a deeply orange/red color was formed. The sol was stable for at least three days before the concentration and purification by dialysis. The spherically shaped particles had an average diameter of 70 nm.

## Example 4

0.1 g $NaBH_4$-Alox (sodium borohydride on alumina, $NaBH_4$ content 10% w/w, Janssen Chimica) was added at room temperature under vigorous stirring to a solution of 0.08 g $SeO_2$ (ICN Chemicals) in 500 ml demineralized water. Immediately an orange/red sol was formed, which, after 5 min, had a stable optical density. After filtration of the Alox particles which are insoluble in water, an orange/red sol, which was stable for at least four days, remained.

## Example 5

80 mg telluric acid $(H_6TeO_6$, Merck) was dissolved in 1 liter of demineralized water. After stirring for 5 minutes at room temperature, a freshly prepared solution of 0.20 g $NaBH_4$ (Chemetal) in 2 ml demineralized water was quickly added under vigorous stirring. After 1 minute, a clear purple/brown sol was formed which was stable in optical density after 3 minutes.

The resulting stable tellurium sol was purified and eventually concentrated by dialysis.

## Claims

1. A process for the preparation of a sol of a non-metallic element selected from the group consisting of selenium and tellurium, comprising the step of treating a solution of a chemical compound, which contains said element in a reducible state, with a reducing agent, characterized in that a borane compound is used as the reducing agent.

2. A process according to claim 1, characterized in that a borane compound, selected from the group consisting of alkali metal borohydrides, alkali metal cyanoborohydrides, quaternary ammonium borohydrides and amine boranes, is used as the reducing agent.

3. A process according to claim 1, characterized in that a borane compound, selected from the group consisting of lithium borohydride ($LiBH_4$), sodium borohydride ($NaBH_4$), potassium borohydride ($KBH_4$), rubidium borohydride ($RbBH_4$), cesium borohydride ($CsBH_4$), lithium cyano borohydride ($LiBH_3CN$), sodium cyano borohydride ($NaBH_3CN$), potassium cyano borohydride ($KBH_3CN$), rubidium cyano borohydride ($RbBH_3CN$), cesium cyano borohydride ($CsBH_3CN$), ammonium borohydride ($NH_4BH_4$), tetramethylammonium borohydride $[(CH_3)_4NBH_4]$, dimethylamino borane $[(CH_3)_2NHBH_3]$, N,N-diethylaniline borane $[C_6H_5N(C_2H_5)_2.BH_3]$ and pyridine borane ($C_5H_5N.BH_3$), is used as the reducing agent.

4. A process according to claim 1, characterized in that sodium borohydride ($NaBH_4$), potassium borohydride ($KBH_4$) or pyridine borane ($C_5H_5N.BH_3$) is used as the reducing agent.

5. A process according to any of claims 1 to 4, characterized in that selenium oxide ($SeO_2$) is used as starting material for preparing a sol of the element selenium.

6. A process according to any of claims 1 to 4, characterized in that telluric acid ($H_6TeO_6$) is used as starting material for preparing a sol of the element tellurium.

7. A sol of a non-metallic element selected from the group consisting of selenium and tellurium, which sol is obtained or obtainable by the process of any of claims 1 to 6.

8. The use of a sol of a non-metallic element selected from the group consisting of selenium and tellurium, which sol is obtained or obtainable by the process of any of claims 1 to 6, for labeling a component of the reaction between a specifically-binding substance and a corresponding bindable substance.

9. The use of a sol of a non-metallic element selected from the group consisting of selenium and tellurium, which sol is obtained or obtainable by the process of any of claims 1 to 6, as a label for a component of the reaction between a specifically-binding substance and a corresponding bindable substance in a method for assaying, detecting or measuring the presence and/or quantity of a component of said reaction in a test sample.

## Patentansprüche

1. Verfahren zur Herstellung eines Sols eines nichtmetallischen Elements, ausgewählt aus der Gruppe bestehend aus Selen und Tellur, wobei das Verfahren den Schritt zur Behandlung einer Lösung einer chemischen Verbindung, die das Element in einer reduzierbaren Stufe enthält, mit einem Reduktionsmittel umfaßt, dadurch gekennzeichnet, daß eine Boranverbindung als Reduktionsmittel verwendet wird.

2. Verfahren nach Anspruch 1, dadurch gekennzeichnet, daß eine Boranverbindung, ausgewählt aus der Gruppe bestehend aus Alkalimetallborhydriden, Alkalimetallcyanoborhydriden, quartären Ammoniumborhydriden und Aminboranen als Reduktionsmittel verwendet wird.

3. Verfahren nach Anspruch 1, dadurch gekennzeichnet, daß eine Boranverbindung, ausgewählt aus der Gruppe bestehend aus Lithiumborhydrid ($LiBH_4$), Natriumborhydrid ($NaBH_4$), Kaliumborhydrid ($KBH_4$), Rubidiumborhydrid ($RbBH_4$), Cäsiumborhydrid ($CsBH_4$), Lithiumcyanoborhydrid ($LiBH_3CN$), Natriumcyanoborhydrid ($NaBH_3CN$), Kaliumcyanoborhydrid ($KBH_3CN$), Rubidiumcyanoborhydrid ($RbBH_3CN$), Cäsiumcyanoborhydrid ($CsBH_3CN$), Ammoniumborhydrid ($NH_4BH_4$), Tetramethylammoniumborhydrid $[(CH_3)_4NBH_4]$, Dimethylaminoboran $[(CH_3)_2NHBH_3]$, N,N-Diethylanilinboran $[C_6H_5N(C_2H_5)_2.BH_3]$ und Pyridinboran ($C_5H_5N.BH_3$) als Reduktionsmittel verwendet wird.

4. Verfahren nach Anspruch 1, dadurch gekennzeichnet, daß Natriumborhydrid ($NaBH_4$), Kaliumborhydrid ($KBH_4$) oder Pyridinboran ($C_5H_5N.BH_3$) als Reduktionsmittel verwendet wird.

5. Verfahren nach einem der Ansprüche 1 bis 4, dadurch gekennzeichnet, daß Selenoxid ($SeO_2$) als Ausgangsmaterial zur Herstellung eines Sols des Elements Selen verwendet wird.

6. Verfahren nach einem der Ansprüche 1 bis 4, dadurch gekennzeichnet, daß Tellursäure ($H_6TeO_6$) als Ausgangsmaterial zur Herstellung eines Sols des Elements Tellur verwendet wird.

7. Sol aus einem nichtmetallischen Element, ausgewählt aus der Gruppe bestehend aus Selen und Tellur, wobei das Sol durch ein Verfahren nach einem der Ansprüche 1 bis 6 erhalten wird oder erhältlich ist.

8. Verwendung eines Sols aus einem nichtmetallischen Element, ausgewählt aus der Gruppe bestehend aus Selen und Tellur, wobei das Sol durch ein Verfahren nach einem der Ansprüche 1 bis 6 erhalten wird oder erhältlich ist, zur Markierung einer Reaktionskomponente zwischen einer spezifisch bindenden Substanz und einer daran bindbaren Substanz.

9. Verwendung eines Sols aus einem nichtmetallischen Element, ausgewählt aus der Gruppe bestehend aus Selen und Tellur, wobei das Sol durch ein Verfahren nach einem der Ansprüche 1 bis 6 erhalten wird oder erhältlich ist, als Markierung einer Reaktionskomponente zwischen einer spezifisch bindenden Substanz und einer daran bindbaren Substanz in einem Verfahren zum Test, Nachweis oder Messen der Anwesenheit und/oder Menge der Reaktionskomponente in einer Probe.

**Revendications**

1. Un procédé de préparation d'un sol d'un élément non métallique pris parmi le sélénium et le tellure, procédé selon lequel on traite une solution d'un composé chimique d'un tel élément dans un état réductible avec un agent réducteur, et qui est caractérisé en ce que l'on utilise comme réducteur un composé de borane.

2. Un procédé selon la revendication 1 caractérisé en ce que le composé de borane est pris parmi des borohydrures et cyanoborohydrures de métaux alcalins, borohydrures d'ammonium quaternaires et aminoboranes.

3. Un procédé selon la revendication 1, caractérisé en ce que le composé de borane est pris parmi le borohydrure de lithium ($LiBH_4$), le borohydrure de sodium ($NaBH_4$), le borohydrure de potassium ($KBH_4$), le borohydrure de rubidium ($RbBH_4$), le borohydrure de césium ($CsBH_4$), le cyano-borohydrure de lithium ($LiBH_3CN$), le cyano-borohydrure de sodium ($NaBH_3CN$), le cyano-borohydrure de potassium ($KBH_3CN$), le cyanoborohydrure de rubidium ($RbBH_3CN$), le cyano-borohydrure de césium ($CsBH_3CN$), le borohydrure d'ammonium ($NH_4BH_4$), le borohydrure de tétraméthylammonium [$(CH_3)_4NBH_4$], le diméthylamino-borane [$(CH_3)_2NHBH_3$], le complexe borane-N,N-diéthylaniline [$C_6H_5N(C_2H_5)_2.BH_3$] et le complexe borane-pyridine ($C_5H_5N.BH_3$).

4. Un procédé selon la revendication 1, caractérisé en ce que l'on utilise comme agent réducteur le borohydrure de sodium ($NaBH_4$), le borohydrure de potassium ($KBH_4$), ou le complexe borane-pyridine ($C_5H_5N.BH_3$).

5. Un procédé selon l'une quelconque des revendications 1 à 4, procédé caractérisé en ce que l'on prépare un sol de sélénium en partant d'oxyde de sélénium ($SeO_2$).

6. Un procédé selon l'une quelconque des revendications 1 à 4, caractérisé en ce que l'on prépare un sol de tellure en partant d'acide tellurique ($H_6TeO_6$).

7. Un sol d'un élément non métallique pris dans le groupe du sélénium et du tellure, sol qui a été obtenu ou que l'on peut obtenir par le procédé de l'une quelconque des revendications 1 à 6.

8. L'emploi d'un sol d'un élément non métallique du groupe du sélénium et du tellure, obtenu ou pouvant être obtenu par le procédé de l'une quelconque des revendications de 1 à 6, pour marquer un composant d'une réaction entre une substance de liaison spécifique et une substance liable correspondante.

9. L'emploi d'un sol d'un élément non métallique du groupe du sélénium et du tellure, obtenu ou pouvant être obtenu par le procédé de l'une quelconque des revendications de 1 à 6, comme marqueur d'un composant d'une réaction entre une substance de liaison spécifique et une substance liable correspondante dans une méthode de dosage, de détection ou de mesure de la présence et/ou de la quantité d'un composant de cette réaction dans un échantillon à examiner.